# NEUE EUROPÄISCHE PATENTSCHRIFT

(11) **EP 3 832 291 B2**
(45) Veröffentlichungstag und Bekanntmachung des Hinweises auf die Entscheidung über den Einspruch: **12.02.2025**
(45) Hinweis auf die Patenterteilung: 15.06.2022
(21) Anmeldenummer: 21154581.9
(22) Anmeldetag: 27.02.2012
(51) Int. Cl.: G01N 21/05, A61L 2/08, G02B 6/00

(54) **DURCHSTRÖMBARE MESSZELLE ZUR AUFNAHME VON MESSMITTELN**
PERMEABLE MEASURING CELL FOR RECEIVING MEASURING MEANS
CELLULE DE MESURE À CIRCULATION POUR LA RÉCEPTION D'ÉLÉMENTS DE MESURE

(30) Priorität: 04.03.2011 DE 102011013001
(43) Veröffentlichungstag der Anmeldung: 09.06.2021
(62) Teilanmeldung aus: 12707727.9
(73) Patentinhaber: optek-Danulat GmbH, 45356 Essen (DE)
(72) Erfinder: PLATTE, Daniel, 42555 Velbert (DE); SCHROEREN, Peter, 47906 Kempen (DE); DANULAT, Jürgen, 40822 Mettmann (DE); REESE, Andreas, 45141 Essen (DE)
(74) Vertreter: Schneider, Sascha

(56) Entgegenhaltungen:
- EP-A1- 0 089 157
- EP-A2- 1 418 419
- WO-A1-2010/126692
- DE-A1- 102011 013 001
- DE-A1- 19 532 382
- DE-A1- 2 132 166
- DE-T2- 69 731 000
- GB-A- 2 282 880
- GB-A- 2 446 934
- US-A- 4 462 962

## Beschreibung

Die vorliegende Erfindung betrifft eine durchströmbare Messzelle zur Aufnahme von Messmitteln zur Messung von chemischen und/ oder physikalischen Eigenschaften eines die Messzelle durchströmenden Fluids gemäß Patentanspruch 1 sowie ein System gemäß Patentanspruch 9.

Insbesondere in der Biotechnologie und der Lebensmitteltechnologie sind durchströmbare Messzellen zur Steuerung und Validierung sowie Einhaltung von bestimmten Vorgaben nicht mehr wegzudenken. Anwendungsbeispiele sind die Chromatographie oder Ultrafiltration. Druckschrift GB 2282880 offenbart eine Vorrichtung zur Messung von Eigenschaften einer Flüssigkeit mit einem Kanal, durch welchen mit verschiedenen Sensoren Eigenschaften des Fluids gemessen werden.

Besonders wichtig ist die Genauigkeit der Messzellen bei der Messung und das schnelle Ansprechen, weshalb solche Messzellen bisher aus qualitativ sehr hochwertigen Materialen hergestellt werden, beispielsweise Edelstahl. Ein wichtiger Aspekt besteht auch in der Reinigungsmöglichkeit, zumal die Messzelle häufig inline eingesetzt werden.

Da häufig, insbesondere in der Biotechnologie, sehr teuere Fluide untersucht werden, spielt auch das Volumen der Messzelle sowie entsprechende Toträume eine große Rolle. So besteht die Bestrebung, das Volumen des Messraums der Messzellen möglichst zu reduzieren, um beispielsweise eine Verschleppung bei einem Phasenwechsel und entsprechenden Materialverbrauch der teueren Medien möglichst gering zu halten. Entscheidend ist aber auch die Leerlaufeigenschaft der Messzelle, damit nach Ende des Messvorgangs keine Reste des Fluids mehr im Messraum verbleiben.

Nicht nur die Reinigung spielt eine entscheidende Rolle, sondern auch die Möglichkeit einer Sterilisierung.

Aufgabe der vorliegenden Erfindung ist es daher, eine nach den vorgenannten Vorgaben optimierte durchströmbare Messzelle anzugeben.

Diese Aufgabe wird mit den Merkmalen der Patentansprüche 1 und 9 gelöst.

Vorteilhafte Weiterbildungen der Erfindung sind in den Unteransprüchen angegeben. Bei angegebenen Wertbereichen sollen auch innerhalb der genannten Grenzen liegende Werte als Grenzwerte offenbart gelten und in beliebiger Kombination beanspruchbar sein.

Der Erfindung liegt der Gedanke zugrunde, eine Messzelle anzugeben, in der bei geringst möglichem Volumen des Messraums sowohl eine Messung mit elektromagnetischer Strahlung als auch eine Leitfähigkeitsmessung und/oder eine pH-Messung und/oder eine Temperaturmessung durchführbar sind. Somit werden mindestens zwei Messungen in einem Messraum durch die erfindungsgemäße Ausgestaltung der durchströmbaren Messzelle ermöglicht, wovon eine eine Strahlungsmessung mit elektromagnetischer Strahlung ist. Insbesondere für die Strahlungsmessung ist zur Ermittlung der Wechselwirkung des Fluids mit der elektromagnetischen Strahlung ein gewisser Strahlengang erforderlich, sodass eine Reduzierung des Volumens kaum möglich ist. Die Erfindung besteht daher darin, mindestens eine weitere Messung im selben Messraum vorzusehen, um das bisher erforderliche Volumen für beide Messungen insgesamt sowie die Anzahl der zu verbauenden Messzellen zu reduzieren.

Gemäß einer vorteilhaften Ausführungsform der Erfindung ist vorgesehen, dass die Messzelle zumindest überwiegend, insbesondere zu mindestens 90%, vorzugsweise zu mindestens 95%, aus chemischen Elementen mit einer Ordnungszahl < 17 besteht. Somit ist die Messzelle so weit gammadurchlässig, dass eine vollständige und homogene Beaufschlagung des Messraums mit Gammastrahlen zur Desinfektion ermöglicht wird. Auf diese Weise wird die Herstellung und der Versand beziehungsweise Transport der erfindungsgemäßen Messzellen stark vereinfacht, da die Messzellen im verpackten Zustand mit Gammastrahlen beaufschlagt und entsprechend desinfiziert werden können. Somit kann eine Kontamination bei der Verpackung der Messzellen ausgeschlossen werden und die Verpackung entsprechend kostengünstiger durchgeführt werden.

Indem die Messzelle einen Temperaturmessbereich zur Anordnung, insbesondere zum Anschluss, eines Temperatursensors aufweist, lässt sich zusätzlich und auf einfache Art und Weise eine Temperaturmessung in die Messzelle integrieren.

Die Erfindung geht gemäß einer vorteilhaften Ausführungsform der Erfindung den zum Stand der Technik entgegengesetzten Weg, indem die Messzelle als Einwegmesszelle, insbesondere überwiegend, vorzugsweise im Wesentlichen vollständig, aus Kunststoff ausgebildet ist. Auf diese Weise ist es möglich, die für die Messung erforderlichen, hochwertigen und teueren Messmittel, für die besonders hohe Qualitätsanforderungen gelten, nach jedem Zyklus oder jeweils nach einer bestimmten Zeitdauer oder sogar bei jedem Wechsel eines Fluids, auszutauschen, während die teuren Messmittel weiterverwendet werden können.

Besonders vorteilhaft ist es bei der vorliegenden Erfindung, dass gemäß einer Ausführungsform der Erfindung der, insbesondere überwiegend röhrenförmige, Messraum ein Volumen von weniger als 50ml, insbesondere weniger als 30ml, aufweist. Somit werden auf kleinstem Raum eine Vielzahl von Messungen an dem die Messzelle durchströmenden Fluid ermöglichst und der Materialverbrauch beziehungsweise die Verschleppung beim Phasenwechsel minimiert.

Da die Eintrittsöffnung und die Austrittsöffnung parallel versetzt zueinander verlaufen, lässt sich die Messzelle optimal in bestehende Systeme einbauen. Hierdurch wird außerdem die Montage der Messzelle erleichtert. Ein besonders gutes Strömungsprofil mit optimalem Leerlaufverhalten ist realisierbar, indem die Messzelle derart gestaltet ist, dass das Fluid von der Eintrittsöffnung bis zur Austrittsöffnung genau zwei Krümmungen durchläuft. Die Krümmungen haben einen Krümmungswinkel von etwa 90°. Auf diese Weise werden an der Messzelle mehrere Freiflächen zur Anbringung von Messmitteln geschaffen.

Gemäß einer weiteren, vorteilhaften Ausführungsform der Erfindung ist vorgesehen, dass ein Strahlengang des Strahlungsmessbereichs quer zum Messraum und quer zur Eintrittsöffnung und/oder Austrittsöffnung verläuft. Auf diese Weise wird die Strahlungsmessung mit geringst möglichem Platzbedarf in die Messzelle oder an der Messzelle verwirklicht.

Für die Leitfähigkeitsmessaufnahme und/oder die pH-Messaufnahme gilt erfindungsgemäß, dass diese längs zur Messform und quer zur Eintrittsöffnung und/oder Austrittsöffnung angeordnet ist/sind. Hierdurch lässt sich unter optimaler Platzausnutzung eine Vollintegration der genannten Messmittel bei geringst möglichen Volumen erreichen.

Das erfindungsgemäße System wird dadurch optimiert, indem gemäß einer vorteilhaften Ausführungsform die Messzelle als Einwegmesszelle für einen Messzyklus verwendbar ist beziehungsweise verwendet wird, während die Strahlungsmessmittel und/oder die Leitfähigkeitsmessmittel und/oder die pH-Wert-Messmittel für mehrere Messzyklen verwendbar sind beziehungsweise verwendet werden.

Weitere Vorteile, Merkmale und Einzelheiten der Erfindung ergeben sich aus der nachfolgenden Beschreibung bevorzugter Ausführungsbeispiele sowie anhand der Zeichnungen. Diese zeigen in:
- Figur 1: eine perspektivische Ansicht einer erfindungsgemäßen Messzelle mit Schnittebene A und Schnittebene B,

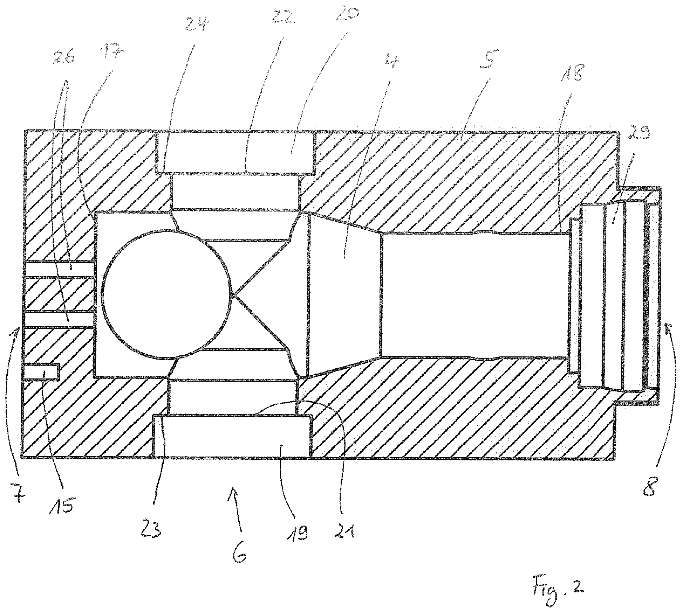

- Figur 2: eine geschnittene Ansicht der Messzelle gemäß Schnittebene A aus Figur 1 und
- Figur 3: eine geschnittene Ansicht der Messzelle der Schnittebene B aus Figur 1.

In den Figuren sind gleiche oder gleich wirkende Elemente oder Elemente mit der gleichen Funktion mit dem gleichen Bezugszeichen gekennzeichnet.

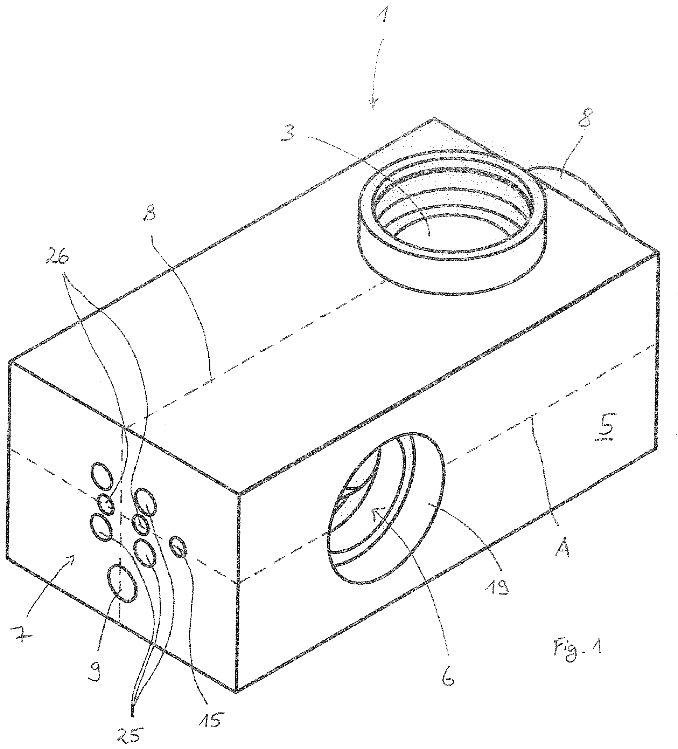

Figur 1 zeigt eine im Wesentlichen quaderförmige, durchströmbare Messzelle 1 mit diversen, unten beschriebenen Mitteln zur Aufnahme von Messmitteln zur Messung von chemischen und/oder physikalischen Eigenschaften eines die Messzelle 1 durchströmenden Fluids.

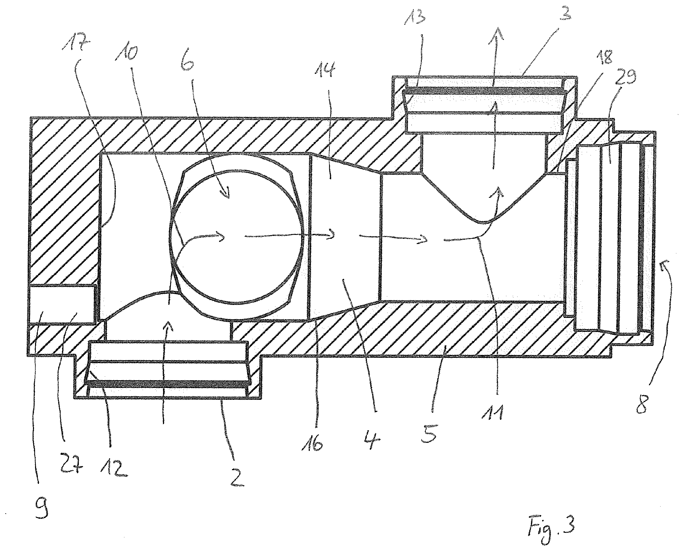

In Figur 3 ist anhand von schematischen Pfeilen (Strömungsverlauf) zu erkennen, dass das Fluid durch eine Eintrittsöffnung 2 zum Einlauf des Fluids in einen Messraum 4 gelangt. Der Messraum 4 erstreckt sich in einem Winkel von 90° zu der Eintrittsöffnung 2 nach rechts, so dass das Fluid einer Krümmung 10 und somit einer schematisch durch einen Pfeil dargestellten Kurve folgt. Nachdem das Fluid durch den Messraum 4 hindurch geströmt ist, läuft das Fluid durch eine Austrittsöffnung 3 aus der Messzelle 1 heraus. Kurz vor der Austrittsöffnung 3 ist eine in die entgegengesetzte Richtung der Krümmung 10 verlaufende Krümmung 11 vorgesehen, sodass das Fluid wiederum einer durch einen Pfeil dargestellte Kurve um 90° folgt.

Sowohl an der Eintrittsöffnung 2 als auch an der Austrittsöffnung 3 sind Anschlussmittel 12, 13 vorgesehen, über welche die Messzelle 1 an entsprechende Anschlüsse in den Prozesslauf als inline-Messzelle anschließbar ist. Im mittleren Bereich des Messraums 4 ist eine, insbesondere konusförmige, Reduzierung 14 des Messraums 4 vorgesehen, um eine möglichst blasenfreie oder laminare Strömung des Fluids zu gewährleisten. An den Anschlussmitteln 12, 13 sind Dichtungsmittel vorgesehen. Für die Anschlussmittel 12, 13 sind mit Vorteil, insbesondere als Einwegadapter ausgebildete, Adapter zum, insbesondere steckbaren, Anschluss verschiedener Leitungsanschlüsse vorgesehen. Die Adapter sind insbesondere aus Kunststoff gebildet und werden gleichzeitig mit der Messzelle verpackt und als Messzellenset zur Verfügung gestellt. Ein solches Messzellenset bietet den Vorteil, dass der inline-Einbau problemlos, schnell und sicher an verschiedene Leitungssysteme erfolgen kann und somit auch die Lagerhaltungskosten reduziert werden.

Die Messzelle 1 besteht im Wesentlichen aus einem einteiligen Messzellenkörper 5 aus Kunststoff, insbesondere Polyphenylensulfon. Erfindungsgemäße Eigenschaften des Kunststoffs sind: präzise Bearbeitbarkeit, hohe Steifheit, Gammadurchlässigkeit und hohe Verbrennbarkeit, d.h. zu mindestens 95% der Masse in der Zeit üblichen Prozessen der Hausmüllverbrennung die Gasphase überführbar.

Durch Vorsehen einer Kodierung 15 ist die Messzelle 1, insbesondere automatisch, mit den Messmitteln unter Vermeidung eines verdrehten oder falschen Anschlusses koppelbar. Hierzu sind an der anzuschließenden Leitung oder einer Aufnahme für die Messzelle 1 an der Leitung korrespondierende Koppelmittel vorgesehen.

Die Kodierung 15 oder eine zusätzliche Kodierung umfasst in einer vorteilhaften Ausführungsform der Erfindung eine Parameterkennung für einen oder mehrere Parameter der Messzelle 1. Die Parameterkennung kann aus einer geometrischen Ausbildung der Kodierung 15 oder der zusätzlichen Kodierung bestehen, die von den Koppelmittel oder separaten Erfassungsmitteln erfasst werden. Besonders vorteilhaft ist eine mechanische oder elektronische Parameterkennung. Als elektronische Parameterkennung kommt insbesondere ein Transponder für die Identifizierung mit Hilfe elektromagnetischer Wellen in Frage.

Die Parameter sind insbesondere die Zellkonstante für die Leitfähigkeitsmessung und/oder die optische Pfadlänge der jeweiligen Messzelle 1.

Der Messraum 4 weist einen, insbesondere röhrenförmigen (vorzugsweise mit kreisförmigen Querschnitt), Messkanal 16 auf, der sich quasi über die gesamte Länge der Messzelle 1 erstreckt. An einem ersten Ende 17 des Messkanals 16 ist die Eintrittsöffnung 2 am Messkanal 16 angewinkelt angeordnet, während an einem zweiten Ende 18 des Messkanals 16 die Austrittsöffnung 3 angewinkelt angeordnet ist, und zwar in entgegengesetzte Richtung zur Eintrittsöffnung 2.

Die Axialrichtung der Eintrittsöffnung 2 und die Axialrichtung der Austrittöffnung 3 sind parallel zueinander und verlaufen quer beziehungsweise unter einem Winkel von 90° zur Axialrichtung des Messkanals 16.

Quer beziehungsweise unter einem Winkel von 90° zum Messkanal sowie insbesondere auch quer beziehungsweise unter einem Winkel von 90° zu der Axialrichtung der Eintrittsöffnung 2 beziehungsweise der Austrittsöffnung 3 liegt ein Strahlenmessbereich 6 zur Messung der Wechselwirkung des Fluids in der Messzelle 1 mit einer elektromagnetischen Strahlung. Elektromagnetische Strahlung tritt von einer nicht dargstellten Strahlungsquelle durch eine Strahlungseintrittsöffnung 19 in den Messraum 4 ein und auf der gegenüberliegenden Seite durch eine Strahlungsaustrittsöffnung 20 wieder aus dem Messraum 4 heraus, wo sie auf eine Strahlungsmesseinrichtung trifft. Der Strahlengang verläuft quer beziehungsweise in einem Winkel von 90° zu dem Messkanal 16 und der Eintrittsöffnung 2 beziehungsweise der Austrittsöffnung 3. Die Axialrichtung der zueinander fluchtenden Strahlungseintrittsöffnung 19 und Strahlungseintrittsöffnung 20 deckt sich mit dem Strahlengang.

In der Strahleneintrittsöffnung 19 und in der Strahlenaustrittöffnung 20 sind jeweils Fensteraufnahmen 21, 22 zur Aufnahme von für die elektromagnetische Strahlung der Strahlungsquelle transparenten Fenstern vorgesehen. Die Fenster dichten den Messraum 4 gegenüber der Umgebung ab.

Die Messzelle 4 ist erfindungsgemäß so ausgebildet, dass im Strahlengang zumindest zwischen zwischen der Strahleneintrittsöffnung 19 und der Strahlenaustrittsöffnung 20, insbesondere zwischen den Fenstern, keine die Messung störenden weiteren Bauteile angeordnet sind.

Die optische Pfadlänge, also die Strecke, die die elektromagnetische Strahlung beim Durchtritt durch das Fluid zurücklegt, ergibt sich durch die Anlage der Fenster an Anschlägen 23, 24 der Fensteraufnahme 21, 22.

An dem ersten Ende 17 ist eine Leitfähigkeitmessaufnahme 7 zur Aufnahme von Leitfähigkeitsmessmitteln zur Messung der Leitfähigkeit des Fluids in der Messzelle 1 vorgesehen. Die Leitfähigkeitsmessaufnahme 7 besteht im vorliegenden Fall aus vier Aufnahmeöffnungen 25 für Stromelektroden und zwei zwischen den Aufnahmeöffnungen 25 angeordneten Aufnahmeöffnungen 26 für Spannungselektroden. In den Aufnahmeöffnungen 25, 26 sind die Strom- beziehungsweise Spannungselektroden dichtend aufnehmbar, sodass diese möglichst plan mit dem ersten Ende 17 abschließen oder leicht in den Messraum 4 hineinragen. Die Funktion eines Leitfähigkeitssensors ist in der DE 19946315C2 beschrieben. In einer vorteilhaften Ausführungsform der Erfindung wird der Leitfähigkeitssensor gemäß DE19946315C2 in einer Aufnahmeöffnung zur Aufnahme des Gehäuses 1 des Leitfähigkeitssensors gemäß DE19946315C2 geeignet und in Kombination mit diesem als Erfindung mitoffenbart.

Neben (vorzugsweise an der gleichen Seite der Messzelle 1) der Leitfähigkeitsaufnahme 7 ist eine mechanische Kodierung 15 vorgesehen, die mit einem entsprechenden Stift eine Aufnahmeeinrichtung beziehungsweise Koppeleinrichtung zur Kopplung der Messzelle 1 an der Prozessleitung vorgesehen, wobei an dem Messzellenkörper 5 mehrere, insbesondere asymmetrisch an dem Messzellenkörper 5 verteilte, Kodierungen 15 vorgesehen sein können.

Ebenfalls neben (vorzugsweise an der gleichen Seite der Messzelle 1) der Leitfähigkeitsaufnahme 7 ist ein Temperaturmessbereich 9 in Form eines fast bis zum Messraum 4 reichenden Sacklochs 27 vorgesehen. Das Sackloch 27 endet in unmittelbarer Nähe des ersten Endes 17 und im Bereich der Eintrittsöffnung 2. Zwischen dem Sackloch 27 und dem erstem Ende 17 ist eine dünne Trennwand 28 vorgesehen, durch die eine Messspitze eines Temperaturfühlers hindurch gestochen werden kann, sodass eine verlässliche Messung und gleichzeitig eine gute Abdichtung gegenüber der Umgebung ermöglicht wird.

Am gegenüberliegenden zweiten Ende 18 ist eine pH-Messaufnahme 8 zur Aufnahme von pH-Wert-Messmitteln zur Messung des pH-Werts des Fluids in der Messzelle 1 vorgesehen. Die pH-Messaufnahme 8 umfasst eine Aufnahmeöffnung 29, deren Axialrichtung mit der Axialrichtung des Messkanals 16 fluchtend ausgerichtet ist. Ein in die Aufnahmeöffnung 29 steckbare pH-Elektrode ist somit dichtend gegenüber der Umgebung der Messzelle 1 in den Messraum 4 steckbar und misst den pH-Wert des vorbeiströmenden Fluids.

Eine Spitze der pH-Elektrode ist bei dem erfindungsgemäßen System mit Vorteil derart in der pH-Messaufnahme 8 anbringbar, dass diese mindestens bis unter der Austrittsöffnung 3, insbesondere mindestens bis zur Mitte der Austrittsöffnung 3 in dem Messkanal 16 reicht. Die pH-Elektrode ist an der Aufnahmeöffnung 29 dichtend fixierbar.

Die Messzelle 1 ist erfindungsgemäß so horizontal ausrichtbar, wie es in den Figuren dargestellt ist, so dass die Eintrittsöffnung 2 und/oder die Austrittsöffnung 3 mit der Normalen fluchten. Hierdurch wird ein optimales Leerlaufverhalten erreicht.

Der Bauraum der Messzelle 1 wird weiter minimiert, wenn dabei ein Strahlengang für die Messung der elektromagnetischen Strahlung (Strahlungsmessbereich 6) horizontal verläuft.

Noch weiter optimierbar ist dies, indem die pH-Messaufnahme im Wesentlichen horizontal, insbesondere maximal mit einem Winkel von 20° zur Horizontalen, verläuft.

### Bezugszeichenliste

1 Messzelle
2 Eintrittsöffnung
3 Austrittsöffnung
4 Messraum
5 Messzellenkörper
6 Strahlungsmessbereich
7 Leitfähigkeitsmessaufnahme
8 pH-Messaufnahme
9 Temperaturmessbereich
10 Krümmung
11 Krümmung
12 Anschlussmittel
13 Anschlussmittel
14 Reduzierung
15 Kodierung
16 Messkanal
17 Erstes Ende
18 Zweites Ende
19 Strahlungseintrittsöffnung
20 Strahlungsaustrittsöffnung
21 Fensteraufnahme
22 Fensteraufnahme
23 Anschläge
24 Anschläge
25 Aufnahmeöffnungen
26 Aufnahmeöffnungen
27 Sackloch
28 Trennwand
29 Aufnahmeöffnung

## Patentansprüche

1. Durchströmbare Messzelle zur Aufnahme von Messmitteln zur Messung von chemischen und/oder physikalischen Eigenschaften eines die Messzelle (1) durchströmenden Fluids mit:
- einer Eintrittsöffnung (2) zum Einlauf des Fluids,
- einer Austrittsöffnung (3) zum Auslauf des Fluids,
- einem, insbesondere einzigen, zwischen der Eintrittsöffnung (2) und der Austrittsöffnung (3) angeordneten Messraum (4),
- einem Strahlungsmessbereich (6) zur Messung der Wechselwirkung des Fluids in der Messzelle (1) mit einer elektromagnetischen Strahlung von außerhalb der Messzelle (1) und
- einer Leitfähigkeitsmessaufnahme (7) zur Aufnahme von Leitfähigkeitsmessmitteln zur Messung der Leitfähigkeit des Fluids in der Messzelle (1) und/oder einer pH-Messaufnahme (8) zur Aufnahme von pH-Wert-Messmitteln zur Messung des pH-Werts des Fluids in der Messzelle (1), **dadurch gekennzeichnet, dass** die Eintrittsöffnung (2) und die Austrittsöffnung (3) parallel versetzt zueinander verlaufen,
wobei das Fluid von der Eintrittsöffnung (2) bis zur Austrittsöffnung (3) genau zwei Krümmungen (10, 11) mit einem Krümmungswinkel von etwa 90° durchläuft,
wobei die Leitfähigkeitsaufnahme (7) und/oder die pH-Messaufnahme (8) längs zum Messraum (4) und quer zur Eintrittsöffnung (2) und/ oder der Austrittsöffnung (3) angeordnet ist/sind.

2. Messzelle nach Anspruch 1, bei der die Messzelle (1) zumindest überwiegend, insbesondere zu mindestens 90%, vorzugsweise zu mindestens 95%, aus chemischen Elementen mit einer Ordnungszahl <17 besteht.

3. Messzelle nach einem der vorhergehenden Ansprüche, bei dem die Messzelle (1) als Einwegmesszelle, insbesondere überwiegend, vorzugsweise im Wesentlichen vollständig, aus Kunststoff ausgebildet ist.

4. Messzelle nach einem der vorhergehenden Ansprüche, bei der die Messzelle einen Temperaturmessbereich (9) zur Anordnung, insbesondere zum Anschluss, eines Temperatursensors aufweist.

5. Messzelle nach einem der vorhergehenden Ansprüche, bei der, der insbesondere überwiegend röhrenförmige, Messraum (4) ein Volumen von weniger als 50ml, insbesondere weniger als 30ml, aufweist.

6. Messzelle nach einem der vorhergehenden Ansprüche, bei der ein Strahlengang des Strahlungsmessbereichs (6) quer zum Messraum (4) und quer zur Eintrittsöffnung (2) und /oder Austrittsöffnung (3) verläuft.

7. Messzelle nach einem der vorhergehenden Ansprüche, bei der die elektromagnetische Strahlung durch eine Strahlungseintrittsöffnung (19) in den Messraum (4) ein und auf der gegenüberliegenden Seite durch eine Strahlungsaustrittsöffnung (20) wieder aus dem Messraum (4) austritt.

8. Messzelle nach Anspruch 7, bei der in der Strahleneintrittsöffnung (19) und in der Strahlenaustrittöffnung (20) jeweils Fensteraufnahmen (21, 22) zur Aufnahme von für die elektromagnetische Strahlung der Strahlungsquelle transparenten Fenstern vorgesehen sind.

9. System aus einer Messzelle nach einem der vorhergehenden Ansprüche und
- einem an der Messzelle anbringbaren Strahlungsmittel zur Erzeugung der elektromagnetischen Strahlung zur Messung der Wechselwirkung des Fluids in der Messzelle (1) mit einer elektromagnetischen Strahlung von außerhalb der Messzelle (1) und
- dem an der Leitfähigkeitsaufnahme (7) anbringbaren Leitfähigkeitsmessmittel und/oder
- dem an der pH-Messaufnahme (8) anbringbaren pH-Wert-Messmittel.

10. System nach Anspruch 9, das eine an einer dem Strahlungsmittel gegenüberliegenden Seite der Messzelle anbringbare Strahlungsmesseinrichtung aufweist.

## Claims

1. Flow-through measurement cell for receiving measurement means for measuring chemical and/or physical properties of a fluid flowing through the measurement cell (1):
- an inlet opening (2) for the inlet of the fluid,
- an outlet opening (3) for the outlet of the fluid,
- a, in particular sole, measurement chamber (4) arranged between the inlet opening (2) and the outlet opening (3),
- a radiation measurement area (6) for measuring the interaction of the fluid in the measurement cell (1) with an electromagnetic radiation from outside the measurement cell (1) and
- a conductivity measurement receptacle (7) for receiving conductivity measurement means for measuring the conductivity of the fluid in the measurement cell (1) and/or a pH measurement receptacle (8) for receiving pH-value measurement means for measuring the pH value of the fluid in the measurement cell (1),
**characterized in that** the inlet opening (2) and the outlet opening (3) are offset in relation to each other in a parallel direction, wherein from the inlet opening (2) to the outlet opening (3) the fluid passes through exactly two, bends (10, 11) with an angle of curvature of 90°,
wherein the conductivity receptacle (7) and/or the pH measurement receptable (8) is/are arranged longitudinally to the measurement chamber (4) and transversely to the inlet opening (2) and/or the outlet opening (3).

2. The measurement cell according to claim 1, wherein at least most, in particular at least 90%, preferably at least 95%, of the measurement cell (1) is composed of chemical elements with an atomic number <17.

3. The measurement cell according to one of the preceding claims, wherein the measurement cell (1) in the form of a disposable measurement cell is composed in particular mostly, preferably essentially entirely, of plastic.

4. The measurement cell according to one of the preceding claims, wherein the measurement cell has a temperature measurement area (9) for arranging, in particular connecting, a temperature sensor.

5. The measurement cell according to one of the preceding claims, wherein the, in particular predominantly tubular, measurement chamber (4) has a volume of less than 50 ml, in particular less than 30 ml.

6. The measurement cell according to one of the preceding claims, wherein a radiation path of the radiation measurement area (6) runs transversely to the measurement chamber (4) and transversely to the inlet opening (2) and/or outlet opening (3).

7. The measurement cell according to one of the preceding claims, wherein the electromagnetic radiation enters the measurement chamber (4) through a radiation inlet opening (19) and exits the measurement chamber (4) on the opposite side through a radiation outlet opening (20).

8. The measurement cell according to claim 7, wherein window receptacles (21, 22) for receiving windows transparent to the electromagnetic radiation of the radiation source are respectively provided in the radiation inlet opening (19) and in the radiation outlet opening (20).

9. System comprising a measurement cell according to one of the preceding claims and
- a radiation means attachable to the measurement cell for generating the electromagnetic radiation for measuring the interaction of the fluid in the measurement cell (1) with an electromagnetic radiation from outside the measurement cell (1) and
- the conductivity measurement means attachable to the conductivity receptacle (7) and/or
- the pH-value measurement means (8) attachable to the pH measurement receptacle.

10. The system according to claim 9, comprising a radiation measurement device attachable to an opposite side of the measurement cell relative to the radiation means.

## Revendications

1. Cellule de mesure perméable pour recevoir des moyens de mesure pour mesurer des propriétés chimiques et/ou physiques d'un fluide traversant la cellule de mesure (1) comprenant :
- une ouverture d'entrée (2) pour l'entrée du fluide,
- une ouverture de sortie (3) pour la sortie du fluide,
- un espace de mesure (4), en particulier unique, disposé entre l'ouverture d'entrée (2) et l'ouverture de sortie (3),
- une zone de mesure de rayonnement (6) pour mesurer l'interaction du fluide dans la cellule de mesure (1) avec un rayonnement électromagnétique venant de l'extérieur de la cellule de mesure (1) et
- une réception de mesure de conductivité (7) pour recevoir des moyens de mesure de conductivité pour mesurer la conductivité du fluide dans la cellule de mesure (1) et/ou une réception de mesure de pH (8) pour recevoir des moyens de mesure de valeur de pH pour mesurer la valeur de pH du fluide dans la cellule de mesure (1),
**caractérisé en ce que** l'ouverture d'entrée (2) et l'ouverture de sortie (3) sont parallèles et décalées entre elles,
et que le fluide, de l'ouverture d'entrée (2) à l'ouverture de sortie (3), traverse précisément deux courbures (10, 11) avec un angle de courbure d'environ 90°,
et que la réception de mesure de conductivité (7) et/ou la réception de mesure de pH (8) est/sont disposée(s) le long de l'espace de mesure (4) et transversalement à l'ouverture d'entrée (2) et/ou l'ouverture de sortie (3).

2. Cellule de mesure selon la revendication 1, dans laquelle la cellule de mesure (1) est composée principalement, en particulier à au moins 90 %, de préférence à au moins 95 %, d'éléments chimiques ayant un nombre atomique < 17.

3. Cellule de mesure selon l'une des revendications précédentes, dans laquelle la cellule de mesure (1) est conçue en tant que cellule de mesure à usage unique, en particulier principalement, de préférence essentiellement totalement en plastique.

4. Cellule de mesure selon l'une des revendications précédentes, dans laquelle la cellule de mesure présente une zone de mesure de températures (9) pour agencer, en particulier pour raccorder, un capteur de mesure.

5. Cellule de mesure selon l'une des revendications précédentes, dans laquelle l'espace de mesure (4) en particulier principalement tubulaire, présente un volume inférieur à 50 ml, en particulier inférieur à 30 ml.

6. Cellule de mesure selon l'une des revendications précédentes, dans laquelle un trajet de faisceau de la zone de mesure de rayonnement (6) est transversal à l'espace de mesure (4) et transversal à l'ouverture d'entrée (2) et/ou l'ouverture de sortie (3).

7. Cellule de mesure selon l'une des revendications précédentes, dans laquelle le rayonnement électromagnétique pénètre dans l'espace de mesure (4) par une ouverture d'entrée de rayonnement (19) et sort de nouveau de l'espace de mesure (4) sur le côté opposé par une ouverture de sortie de rayonnement (20).

8. Cellule de mesure selon la revendication 7, dans lequel sont prévues dans l'ouverture d'entrée de rayonnement (19) et dans l'ouverture de sortie de rayonnement (20), des réceptions de fenêtre (21, 22) respectives pour recevoir des fenêtres transparentes pour le rayonnement électromagnétique de la source de rayonnement.

9. Système composé d'une cellule de mesure selon l'une des revendications précédentes et
- d'un moyen de rayonnement pouvant être appliqué sur la cellule de mesure pour produire le rayonnement électromagnétique pour mesurer l'interaction du fluide dans la cellule de mesure (1) avec un rayonnement électromagnétique venant de l'extérieur de la cellule de mesure (1) et
- du moyen de mesure de conductivité pouvant être appliqué sur la réception de mesure de conductivité (7) et/ou
- du moyen de mesure de valeur de pH pouvant être appliqué sur la réception de mesure de pH (8).

10. Système selon la revendication 9, qui présente un dispositif de mesure de rayonnement pouvant être appliqué sur un côté de la cellule de mesure opposé au moyen de rayonnement.
